# EUROPÄISCHE PATENTSCHRIFT

(11) **EP 2 822 958 B1**
(45) Veröffentlichungstag und Bekanntmachung des Hinweises auf die Patenterteilung: **19.12.2018**
(21) Anmeldenummer: 13708150.1
(22) Anmeldetag: 07.03.2013
(51) Int. Cl.: G01N 33/98

(54) **ANTIMIKROBIELLE PEPTIDE**
ANTIMICROBIAL PEPTIDES
PEPTIDES ANTIMICROBIENS

(30) Priorität: 07.03.2012 DE 102012203547
(43) Veröffentlichungstag der Anmeldung: 14.01.2015
(73) Patentinhaber: Robert Bosch Gesellschaft für medizinische Forschung mbH, 70376 Stuttgart (DE)
(72) Erfinder: WEHKAMP, Jan, 70372 Stuttgart (DE); SCHROEDER, Bjoern, 70376 Stuttgart (DE); STANGE, Eduard, 70469 Stuttgart (DE)
(74) Vertreter: Bee, Joachim
(86) Internationale Anmeldenummer: PCT/EP2013/054599
(87) Internationale Veröffentlichungsnummer: WO 2013/132005

(56) Entgegenhaltungen:
- EP-A1- 2 077 274
- WO-A1-2012/028479
- WO-A2-2009/117524
- US-B1- 8 071 285
- NATACHA FRISON ET AL: "Oligolysine-based saccharide clusters: synthesis and specificity", BIOCHEMICAL JOURNAL, Bd. 368, Nr. 1, 15. November 2002 (2002-11-15), Seite 111, XP055061063, ISSN: 0264-6021, DOI: 10.1042/BJ20020673
- BJOERN O. SCHROEDER ET AL: "Reduction of disulphide bonds unmasks potent antimicrobial activity of human [beta]-defensin 1", NATURE, Bd. 469, Nr. 7330, 20. Januar 2011 (2011-01-20), Seiten 419-423, XP55016876, ISSN: 0028-0836, DOI: 10.1038/nature09674
- OLGA SCUDIERO ET AL: "Novel synthetic, salt-resistant analogs of human beta-defensins 1 and 3 endowed with enhanced antimicrobial activity", ANTIMICROBIAL AGENTS AND CHEMOTHERAPY, AMERICAN SOCIETY FOR MICROBIOLOGY, US , vol. 54, no. 6 1 June 2010 (2010-06-01), pages 2312-2322, XP002694699, ISSN: 0066-4804, DOI: 10.1128/AAC.01550-09 Retrieved from the Internet: URL:http://aac.asm.org/content/54/6/2312 [retrieved on 2010-03-22]

## Beschreibung

Die vorliegende Erfindung betrifft neue antimikrobielle Peptide sowie deren Verwendung in der Medizin.

Antimikrobielle Peptide, die auch kurz mit "AMP" bezeichnet werden, sind Teil des angeborenen Immunsystems und lebenswichtig für die epitheliale Abwehr gegenüber Infektionen von Mikroorganismen.

Bei einem gesunden Menschen bilden Haut und Schleimhaut eine physikalische Barriere gegenüber einer Infektion durch Mikroorganismen. Die physikalische Barriere besteht in der gesunden Haut aus dem *Stratum corneum* und in der Schleimhaut aus der Schleimschicht, in welchen Desquamation und Schleimhautabsonderung zur ständigen Erneuerung der Oberflächen führt, sowie gleichzeitig zu einer kontinuierlichen Elimination von Mikroorganismen, die an den Oberflächen anhaften. In Zusammenwirkung mit den in der Haut ferner vorliegenden Lipiden verhindert diese physikalische Barriere, dass Mikroorganismen in die lebende Epidermis eindringen.

Abgesehen von dieser physikalischen Barriere sind für eine erfolgreiche Infektionsabwehr der gesunden Haut und Schleimhaut jedoch noch weitere Faktoren notwendig, zu denen u.a. körpereigene antimikrobielle Peptide zählen. So ist Beispiels Lysozym ein antimikrobielles Peptid, das in Nasensekret vorliegt und das insbesondere grampositive Bakterien abtöten kann. Ferner sind in der Darmmukosa die Defensine als antimikrobielle Peptide bekannt, deren Vorliegen insbesondere vor dem Hintergrund notwendig erscheint, dass die Darmepitelien in extrem hohen Mengen Bakterien ausgesetzt sind. Neben der für Mikroorganismen nur schwer zu durchdringenden Schleimschicht sind in der Schleimhaut des Darmes Paneth-Zellen zu finden, die das humane Defensin-5 sezernieren und die u.a. die Stammzellen schützen, die für die ständige Erneuerung der Darmschleimhaut wichtig sind.

Weitere bekannte AMP sind ein als Psoriasin bekanntes Peptid, sowie die RNase-7, die ein wirksames, körpereigenes Breitbandantibiotikum des Menschen darstellt.

Neben den bekannten körpereigenen antimikrobiellen Peptiden sind im Stand der Technik daneben noch zahlreiche Antibiotika bekannt, wobei hierunter sowohl Substanzen biologischen Ursprungs als auch synthetisch hergestellte Substanzen fallen, die also entweder - wie im ursprünglichen Sinne - natürlich gebildete niedermolekulare Stoffwechselprodukte von Pilzen oder Bakterien sind, oder aber chemisch synthetisierte Therapeutika.

Insbesondere vor dem Hintergrund, dass die Entwicklung von Resistenzen gegenüber natürlichen und synthetischen Antibiotika die Behandlung von mikrobiellen Infektionskrankheiten zunehmend erschwert wird, besteht auch regelmäßig das Bedürfnis an neuen antimikrobiellen Wirkstoffen, die sich durch geringe Nebenwirkungen, einfache Herstellung und Handhabung auszeichnen

Vor diesem Hintergrund ist es Aufgabe der vorliegenden Erfindung, eine neue antimikrobielle Substanz bereitzustellen, die zur Behandlung von infektiösen mikrobiellen Erkrankungen eingesetzt werden kann.

Erfindungsgemäß wird diese Aufgabe durch ein Peptid gelöst, das antimikrobielle Aktivität aufweist, und einen C- und einen N-Terminus aufweist, und das aus acht aufeinander folgenden Aminosäuren besteht, wobei das Peptid eine Sequenz mit der folgenden Formel I besitzt:

Ter₁-X₁-B₁-X₂-B₂-X₃-Z₁-Z₂-X₄-Ter₂, (Formel I)

in welcher
Ter₁ die freie N-terminale Aminogruppe der N-terminalen Aminosäure X₁ ist, oder eine modifizierte N-terminale Aminogruppe;
X₁, X₂ und X₃ jeweils gleich oder unterschiedlich sind und unabhängig voneinander jeweils ausgewählt sind aus einer Aminosäure mit einer basischen Seitenkette, vorzugsweise ausgewählt sind aus einem der folgenden: Arginin, Lysin, 6-Hydroxylysin, Homoarginin, 2,4-Diaminobuttersäure, [beta]-Homoarginin, D-Arginin, Arginal, 2-Amino-3-guanidinopropionsäure, Nitroarginin, N-Methyl-Arginin, [epsilon]-N-Methyllysin, allo-Hydroxylysin, 2,3-Diaminopropionsäure, 2,2'-Diaminopimelinsäure, Ornithin, sym-Dimethylarginin, asym-Dimethylarginin,
B₁ und B₂ gleich oder unterschiedlich sind und jeweils unabhängig voneinander jeweils ausgewählt sind aus einer Aminosäure mit einer aliphatischen oder basischen Seitenkette, und vorzugsweise ausgewählt sind aus Alanin oder Glycin,
Z₁ und Z₂ jeweils Cystein und Alanin sind und
Ter₂ die freie C-terminale Carboxylgruppe der C-terminalen Aminosäure X₄ ist, oder eine modifizierte C-terminale Carboxylgruppe.

Z₁ und Z₂ sind, wie bereits aufgeführt dabei jeweils Cystein und Alanin, d.h. wenn Z₁ Cystein ist, dann ist Z₂ Alanin, und wenn Z₁ Alanin ist, ist Z₂ Cystein.

EP 2 077 274 A1 offenbart von hBD1 und hBD3 abgeleitete antimikrobielle Peptide sowie deren pharmazeutische und medizinische Verwendung. Bevorzugt sind Peptide der Sequenzen, die das Sequenzmotiv gemäß Formel I aufweisen sowie Fragmente davon. Darüber hinaus wird die Substitution von L-durch R-Aminosäuren sowie Modifikation der freien N-/C-Termini gezeigt.

Aus Bjoern O. Schroeder et al "Reduction of disulphide bonds unmasks potent antimicrobial activity of human [beta]-defensin 1", Nature, Bd. 469, Nr.7330, 20. Januar 2011, Seiten 419-423, XP055016876 ist bekannt, dass das C-terminale hBD1 Heptapeptid GKAKCCK antimikrobielle Aktivität aufweist, die insbesondere dem Vorhandensein zweier freien Cysteinreste zugeschrieben wird.

Auch bei Olga Scudiero et al: "Novel synthetic, salt resistant analogs of human [beta]-defensin 1 and 3 endowed with enhanced antimicrobial activity, Antimicrobial agents and chemotherapy, Bd. 54, Nr. 6, 1. Juni 2010, Seiten 2312-2322, XP002694699 wird die salzunabhängige antimikrobielle Aktivität dem hBD3 C-Terminus zugeschrieben.

Die US 8071285 B1 offenbart Zink-Finger-Proteine, die an bestimmte zelluräre Zielsequenzen binden und dort die Transkriptionsfunktion der zellulären Zielsequenzen modulieren können. U.a. wird die 10 Aminosäuren umfassende Sequenz TCYRGKAKCC (SEQ ID Nr. 3) offenbart. Diese Sequenz weist in der Sequenz RGKAKCC eine übereinstimmende Aminosäuren-Abfolge auf.

Das Peptid ist dabei vorzugsweise synthetisch hergestellt, rekombinant hergestellt, durch enzymatische Spaltung gewonnen und/oder isoliert. Da es sich bei dem erfindungsgemäßen Peptid um ein relativ kurzes Peptid handelt, ist bevorzugt, wenn das erfindungsgemäße Peptid synthetisch hergestellt ist; synthetische Herstellungsverfahren sind im Stand der Technik hinreichend bekannt, und umfassen insbesondere Flüssigphasen und Festphasenchemische Syntheseverfahren. Beispielhaft wird auf den Übersichtsartikel /das Standardwerk Kent, S., "Chemical Synthesis of Peptides and Proteins", Annual Review of Biochemistry 57:957-989 (1988) verwiesen. Im betreffenden Gebiet sind gegenwärtig auch zahlreiche Firmen tätig, die synthetische Peptide kommerziell herstellen.
vorliegend werden hierunter insbesondere Bakterien, Viren und Pilze verstanden, die in anderen Organismen, insbesondere dem Menschen oder anderen Säugetieren, gesundheitsschädigende Abläufe (Erkrankungen) verursachen.

Gemäß einer bevorzugten Ausführungsform ist das erfindungsgemäße Peptid dabei ausgewählt aus einer der SEQ ID Nr. 2, 3, 5 oder 6, oder Derivaten davon, wobei die Derivate durch Austausch zumindest einer Aminosäure durch ein Derivat der Aminosäure gebildet sind. Dabei sind insbesondere die folgenden Peptide bevorzugt: das Peptid mit der Sequenz RGKAKCAK (SEQ ID Nr. 2) und das Peptid mit der Sequenz RGKAKACK (SEQ ID Nr. 3) bevorzugt, und zwar in unmodifizierter Form, d.h. mit unmodifizierten Termini oder in modifizierter Form, d.h. mit zumindest einem modifizierten (C- oder N-)- Terminus oder in modifizierter Form mit einem modifizierten N- und einem modifizierten C-Terminus (siehe die SEQ ID Nrn. 5 und 6).

Unter dem Begriff "Derivat der/einer Aminosäure" sind dabei alle von der jeweiligen Aminosäure abgeleiteten Aminosäurereste zu verstehen, die aus der jeweiligen Aminosäure bspw. durch strukturelle Veränderung einer funktionellen Gruppe erhalten werden.

Unter dem Begriff "Modifizierte N-terminale Aminogruppe" und "modifizierte C-terminale Carboxylgruppe" wird vorliegend eine veränderte Aminogruppe oder Carboxygruppe verstanden. Beispiele für N-terminale Modifizierungen sind acetylierte, formylierte oder guanylierte N-Termini. Beispiele für C-terminale Modifizierungen sind amidierte C-Termini.

Dabei ist insbesondere bevorzugt, wenn das Peptid jeweils insgesamt aus D-Aminosäuren oder L-Aminosäuren oder aus Mischungen davon besteht. Dabei bedeutet vorliegend "D-Aminosäuren" oder "L-Aminosäuren", dass die einzusetzenden natürlichen Aminosäuren, unnatürlichen Aminosäuren oder Aminosäurederivate (wie Iminosäuren) in der L- oder der D-Konfiguration vorliegen können.

Gemäß einer weiteren Ausführungsform ist bevorzugt, wenn das Peptid am C-Terminus und/oder am N-Terminus modifiziert ist, und insbesondere durch eine Acetylierung, Amidierung, Formylierung oder Guanylierung modifiziert ist.

Die Modifizierung der C- und/oder N-Termini der erfindungsgemäßen Peptide hat den Vorteil, das diese dadurch stabiler gegenüber dem Abbau durch Peptidasen und Proteasen sind; damit besitzen die erfindungsgemäßen Peptide eine erhöhte Halbwertszeit in bspw. Serum. Die Modifizierungen der N- und C-Termini erlauben auch das Koppeln der Peptide an andere Gruppen, wie zum Beispiel andere Aminosäuresequenzen oder andere Biomoleküle.

In einer weiteren Ausführungsform des erfindungsgemäßen Peptids ist dieses reduziert oder liegt in oxidiertem Zustand vor.

Erfindungsgemäß wird das Peptid zur Behandlung und/oder Prophylaxe von entzündlichen oder infektiösen Erkrankungen, die durch Mikroorganismen ausgelöst werden, verwendet.

Erfindungsgemäß erfolgt die Verwendung daher bei entzündlichen und/oder infektiösen Erkrankungen, die durch Bakterien, Viren oder Pilze verursacht werden.

Insbesondere erfolgt die erfindungsgemäße Verwendung bei entzündlichen oder infektiösen Erkrankungen, die durch einen Mikroorganismus verursacht sind, der ausgewählt ist *aus Bifidobakterium* sp., *Lactobacillus* sp., *Escherichia coli, Streptococcus* sp., *Staphylococcus* sp., *Bacteroides* sp., *Candida* sp., *Pseudomonas sp., Propionibacterium sp., Treponema sp., Enterobacter sp., Salmonella sp., Legionella sp.,* wobei sich versteht, dass diese Aufzählung nicht abschließend ist, und das Peptid auch wirksam gegen hierin nicht aufgeführte Bakterien- und/oder Pilzstämmen ist, und insbesondere gegen Bakterien, die allgemein zur Familie der Neisseriaceae, Enterobacteriaceae, gehören.

Dabei ist insbesondere bevorzugt, wenn die Verwendung des erfindungsgemäßen Peptides bei chronischen entzündlichen Darmerkrankungen, entzündlichen Erkrankungen des Mund-Rachenraumes, wie bspw. Karies und Zahnfleischentzündlichen, Lungenerkrankungen, Erkrankungen des Urogenitaltraktes, Erkrankungen der Bauchspeicheldrüse, Erkrankungen des weiblichen Reproduktionstraktes, Erkrankungen und/oder Verletzungen der Haut (dermatologische Erkrankungen) erfolgt.

Entsprechend betrifft die vorliegende Erfindung auch eine pharmazeutische Zusammensetzung, die zumindest ein erfindungsgemäßes Peptid aufweist, sowie ggf. einen pharmazeutisch akzeptierbaren Träger und weitere im Stand der Technik üblichen Formulierungs- und Hilfsstoffe, sowie ein Verfahren zur Behandlung von Säugetieren, die an entzündlichen infektiöse Erkrankungen, ausgelöst durch Mikroorganismen, leiden, bei welchem eine therapeutisch effektive Menge an dem erfindungsgemäßen Peptid oder der erfindungsgemäßen pharmazeutischen Zusammensetzung verabreicht wird. Dabei bedeutet "therapeutisch wirksame" oder "therapeutisch effektive Menge" die Menge des zumindest einen erfindungsgemäßen Peptids oder der pharmazeutischen Zusammensetzung, die zumindest ein erfindungsgemäßes Peptid aufweist, welche die Vermehrung und Kolonienbildung der Bakterien und/oder Pilze zu reduzieren oder ganz zu verhindern oder einen messbaren therapeutischen bzw. prophylaktischen Erfolg zu erzielen vermag. Die genaue effektive Menge für ein Subjekt hängt von dessen Größe und Gesundheitszustand, der Art und dem Ausmaß der Erkrankung und dem zumindest einen Peptid oder der pharmazeutischen Zusammensetzung oder der Kombination mehrerer der genannten ab.

Die Formulierungen/Arzneimittel der vorliegenden Erfindung können entweder *in vitro* - oder *in vivo* angewendet werden.

Die pharmazeutische Zusammensetzungen der vorliegenden Erfindung können einem Patienten in einer Vielzahl von Formen verabreicht werden, die den gewählten Weg der Verabreichung, nämlich parenteral, oral, intraperitoneal, transdermal etc. angepasst sind. Eine parenterale Verabreichung schließt dabei die Verabreichung auf folgenden Wegen ein: Intravenös, intramuskulär, interstitiell, intraarteriell, subkutan, intrasynovial, transepitelial, einschließlich transdermal, pulmonal via Inhalation, ophthalmisch, sublingual und bukkal, topisch einschließlich ophthalmisch, dermal, okular, rektal sowie eine nasale Inhalation via Insufflation ein.

Die Verabreichung kann dabei in Form von Lösungen, Tinkturen, Salben, Pulvern, Suspensionen, Cremes, und weiteren festen oder flüssigen Formulierungen sowie als Tabletten, Kapseln, Spray, verabreicht werden.

Zu den Erkrankungen der Haut, die mit dem vorliegenden erfindungsgemäßen Peptid, bzw. einem dieses enthaltende Arzneimittel behandelt werden können, zählen beispielsweise Akne, Dermatitis, Verbrennungen, und andere Erkrankungen der Haut, die durch Mikroorganismen ausgelöst wurden, bzw. bei Verletzungen der Haut, bei denen die Gefahr einer mikrobiellen Infektion besteht.

Gemäß einer bevorzugten Ausführungsform wird die pharmazeutische Zusammensetzung durch bzw. über die Haut verabreicht, was eine nicht invasive und patientenfreundliche Verabreichung darstellt, und gegenüber einer oralen Verabreichung den Vorteil hat, dass das Milieu im Verdauungssystem nicht beachtet werden muss. Die Aufnahme durch die Haut ist beispielsweise in der Nase, der Wange, unter der Zunge, am Zahnfleisch oder in der Vagina möglich. Entsprechende Darreichungsformen können mit bekannten Techniken erreicht werden; sie können zu Nasentropfen, Nasenspray, Einlagen, Filmen, Pflaster, Gele, Zäpfchen, Salben oder Tabletten verarbeitet werden. Bevorzugt wird der Arzneiträger für die Aufnahme durch die Haut eine oder mehrere Komponenten enthalten, die an der Haut haften und dadurch die Kontaktzeit der Darreichungsform mit der adsorbierenden Oberfläche verlängern, um dadurch die Aufnahme durch Absorption zu steigern. So kann das zumindest eine erfindungsgemäße Peptid bspw. in Liposomen formuliert sein, die das Einbringen des Peptids in die Haut unterstützen.

Das erfindungsgemäße Peptid kann ferner zur Behandlung von Erkrankungen des Mund-Rachenraumes eingesetzt werden, und kann bei derartigen Einsätzen in Form von Zahncremes, Mundwässern, Gelen, und/oder beispielsweise auf Zahnseide vorliegen.

Die pharmazeutische Zusammensetzung kann, wie bereits zuvor erwähnt, neben dem zumindest einen erfindungsgemäßen Peptid auch zwei oder mehrere der erfindungsgemäßen Peptide enthalten. Ferner kann die pharmazeutische Zusammensetzung neben dem zumindest einen erfindungsgemäßen Peptid auch noch eines oder mehrere weitere aktive Substanzen aufweisen, wie bspw. im Stand der Technik bekannte Antibiotika (z.B. Streptomycin, Penicillin, Tetracyclin) oder andere antimikrobiell aktive Verbindungen wie Fungizide, bspw. Myconazol, oder andere Substanzen, mit welchen die mit einer Infektion üblicherweise einhergehende Symptome behandelt werden, wie bspw. Fieber oder Hautausschlag.

Daneben kann das Arzneimittel auch pharmazeutisch akzeptierbare Träger, Verbindungsmittel, Exzipienten oder Adjuvanzien aufweisen. Die Wahl eines pharmazeutischen Trägers, Exzipienten oder Verdünnungsmittel kann im Hinblick auf den beabsichtigten Verabreichungsweg und die standardisierte pharmazeutische Praxis vorgenommen werden. Als pharmazeutisch akzeptable Träger können Lösungsmittel, Streckmittel oder andere flüssige Bindemittel wie Dispersions- oder Suspensionshilfsmittel, oberflächenaktive Agenzien, isotonische Wirkstoffe, Dickungsmittel oder Emulgatoren, Konservierungsmittel, Umhüllungsmittel (encapsulating agent), feste Bindestoffe oder Gleitmittel eingesetzt werden, je nachdem was für die jeweilige Dosierung am Besten geeignet ist und zugleich mit dem Peptid kompatibel ist. Die pharmazeutische Zusammensetzung kann auch noch Puffer, Verdünnungsmittel und/oder Additive enthalten. Geeignete Puffer schließen bspw. Tris-HCI, Glycin und Phosphat mit ein, und geeignete Verdünnungsmittel bspw. wässrige NaCI-Lösungen, Lactose oder Mannitol. Geeignete Additive schließen bspw. Detergentien, Lösungsmittel, Antioxidantien und Konservierungsstoffe und Schutzkolloide, wie bspw. homologes Albumin oder biokompatible Hydrogele, mit ein. Eine Übersicht über solche zusätzlichen Inhaltsstoffe findet sich bspw. in A. Kibbe.: "Handbook of Pharmaceutical Excipients", 3rd Ed., 2000, American Pharmaceutical Association and Pharmaceutical Press.

Ferner kann die erfindungsgemäße pharmazeutische Zusammensetzung auch pharmazeutisch akzeptable Salze aufweisen, wie z. B. Salze von Mineralsäuren, wie Hydrochloride, Hydrobromide, Phosphate, Sulfate und vergleichbare; aber auch Salze organischer Säuren, wie Acetate, Propionate, Malonate, Benzoate und vergleichbare.

Im Allgemeinen wird eine therapeutisch effektive tägliche Dosis der vermutlich im Bereich von 0,01 bis 50 mg/kg Körpergewicht des zu behandelnden Subjekts, vorzugsweise von 0,1 bis 20 mg/kg, liegen. Wie bereits ebenfalls weiter oben erwähnt, kann das Arzneimittel in Form von Tabletten oder Kapseln bereitgestellt werden, die einzeln oder zwei oder mehrere hiervon zur gleichen Zeit verabreicht werden können. Das Arzneimittel kann auch in Form einer Formulierung mit verzögerter Freisetzung bereitgestellt werden.

Typischerweise wird der Arzt die tatsächliche Dosis bestimmen, die für einen bestimmten Patienten geeignet ist, was in Abhängigkeit seines Alters, des Gewichts und dem allgemeinen Gesundheitszustands des Patienten abhängen wird.

Je nach Anwendung kann das Arzneimittel durch Inhalation, in Form eines Zäpfchens oder Pessar, topisch als Lösung, Lotion, Salbe, Creme oder Streupuder, unter Verwendung eines Hautpflasters, oral in Form von Tabletten, oder Kapseln, Elixieren, Lösungen oder Suspensionen, welche ggf. Aromastoffe oder Färbemittel enthalten, verabreicht werden.

Neben der therapeutischen Verwendung zur Behandlung von Infektionen, kann das zumindest eine erfindungsgemäße Peptid auch in Desinfektions- oder Reinigungsmitteln eingesetzt werden, die zur Desinfektion oder Reinigung von Oberflächen oder Gegenständen verwendet werden können. Ein anderes Anwendungsgebiet sind Verpackungen, wo Peptide an Verpackungsmaterial gebunden oder darin eingebunden werden können, oder als Konservierungsmittel für andere Materialien, die leicht durch Mikroorganismen abgebaut werden können.

Neben der Verwendung des erfindungsgemäßen Peptids in der Humanmedizin ist auch eine Verwendung in der Veterinärmedizin möglich.

Die Erfindung betrifft ferner ein isoliertes Nukleinsäuremolekül, dessen Sequenz für das erfindungsgemäße Peptid codiert, und insbesondere für ein Peptid mit der SEQ ID Nr. 1 bis 6 die für das erfindungsgemäße antimikrobielle, d.h. antibakterielle oder antimykotische, Peptid oder codierende Nukleinsäure steht dabei in operativer Verbindung mit einer regulatorischen Sequenz, die dessen Expression in der Wirtszelle steuert. Ein weiterer Bestandteil der Erfindung ist eine Wirtszelle, die mit dem oben beschriebenen Nukleinsäuremolekül transfiziert oder transformiert ist.

Weitere Vorteile ergeben sich aus der nachstehenden Beschreibung sowie den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

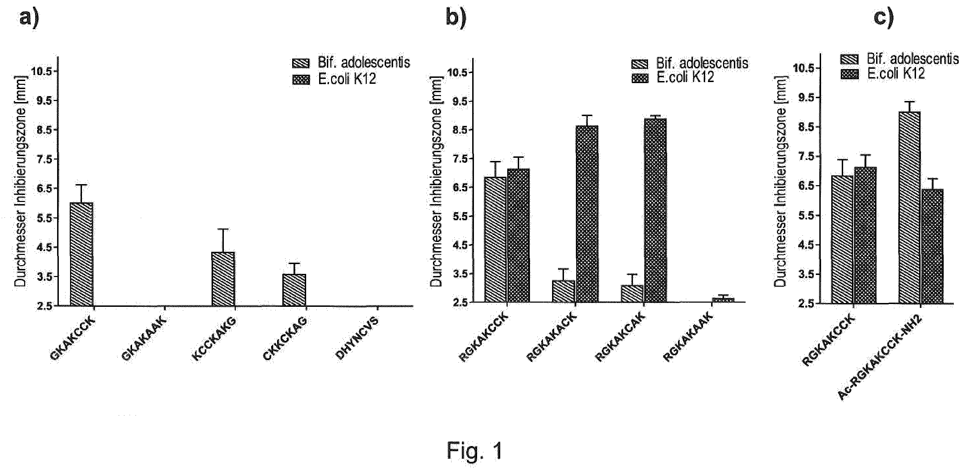

- Fig. 1: die Ergebnisse der Untersuchungen der antimikrobiellen Wirkung verschiedener Peptide (Heptapeptide (a); Octapeptide (b) (c)) in Bezug auf die Bakterien *Bifidobacterium adolescentis* oder *Escherichia coli.* Die Buchstaben geben die Aminosäuren im Einbuchstabencode an. Das Peptid ac-RGKAKCCK-NH₂ (c) (SEQ ID Nr. 4) besitzt einen acetylierten Amino-Terminus und einen amidierten Carboxy-Terminus. Der Durchmesser der Inhibierungszonen repräsentiert die antimikrobielle Aktivität; dabei ist ein Durchmesser von 2,5 mm der Durchmesser eines leeren gestanzten Wells in eine Agarplatte, welches nur Trägerflüssigkeit enthält (Negativkontrolle). Die Versuche wurden mindestens dreimal wiederholt, und der Mittelwert + Standardabweichung ist gezeigt;

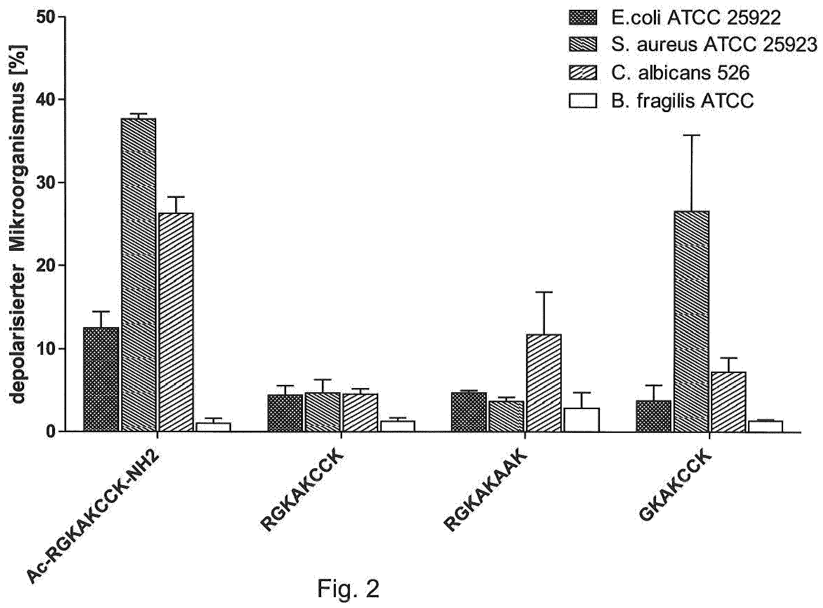

- Fig. 2: die Untersuchung verschiedener Ausführungsformen des Peptids als Antibiotikum gegen unterschiedliche Pathogene: folgende Peptide wurden untersucht: modifiziertes Octapeptid (SEQ ID Nr. 4), Wildtyp Octapeptid (SEQ ID Nr. 1), Alanin-mutiertes Octapeptid (SEQ ID Nr. 7) und ein Heptapeptid (SEQ ID Nr. 8) (jeweils 50 µg/ml) wurden in einem Durchflusszytometrischen-antimikrobiellen Wirksamkeits-Assay gegen *Escherichia coli, Staphylococcus aureus, Candida albicans* und *Bacteroides fragilis* getestet. Die Buchstaben geben wiederum die jeweiligen Aminosäuren im Einbuchstabencode an. Die Versuche wurden zweimal in Doppelansätzen wiederholt, der Mittelwert + Standardabweichung ist gezeigt;
- Fig. 3: die Ergebnisse der Untersuchungen eines erfindungsgemäßen Octapeptids, das aus D-Aminosäuren besteht, im Vergleich zu einem aus L-Aminosäuren bestehenden Octapeptid gegenüber *E. coli K12* (a) bzw. *Bifidobacterium adolescentis* (b);
- Fig. 4: die Ergebnisse weiterer Untersuchungen zur Aktivität verschiedener erfindungsgemäßer Octapeptide gegenüber pathogenen Bakterien und Pilzen im Radial-Diffusionsassay; und
- Fig. 5: die Ergebnisse von Untersuchungen zur Zelltoxizität der Octapeptide gegen die Darmzelllinie CaCo-2.

Wie einleitend ausgeführt werden antimikrobielle Peptide (AMP) von nahezu allen Organismen produziert und stellen eine erste Barriere gegen mikrobielle Infektionen dar. Viele AMPs weisen eine antimikrobielle Aktivität sowohl gegen grampositive als auch gegen gramnegative Bakterien, sowie gegen Pilze und einige Viren mit Hüllen auf. Der Mensch produziert unterschiedliche Klassen an AMPs, wobei eine davon, wie ebenfalls bereits weiter oben erwähnt, die Defensine sind. Diese zeichnen sich durch ihre geringe Größe (3 bis 5 kDa), einer kationischen Nettoladung und sechs konservierte Cysteinreste aus, die über drei Disulfidbrücken miteinander verbunden sind. In Abhängigkeit der Konnektivität dieser Brücken werden die Defensine in Alpha- und Beta-Defensine eingeteilt. Bis heute wurden lediglich vier Beta Defensine (hBD-1 bis hBD-4) funktionell untersucht, u.a. auch als antibiotisch wirksame Kandidaten.

Bis heute stellt jedoch die chemische Synthese von Beta-Defensinen, die, wie weiter oben bereits erwähnt, drei native Disulfidbrücken aufweisen, eine große Herausforderung dar, was sowohl die Kosten als auch den Aufwand des Herstellungsverfahren anbelangt.

Das mit der vorliegenden Erfindung erstmals bereitgestellte Peptid stellt ein Oktopeptid des C-terminalen Endes des Defensins hBD-1 dar, das zwei freie Cysteine enthält, und das sich in seiner antimikrobiellen Aktivität als überragend im Vergleich zu hBD-1, sowie kürzeren Peptidsequenzen aus dem C-Terminus von hBD-1 erwiesen hat, wie die nachfolgend aufgeführten Versuche zeigen.

### Bakterien- und Pilzstämme

Die Bakterienstämme *Bifidobacterium adolescentis* Ni3, 29c (klinisches Isolat), *Bifidobacterium breve* PZ1343, *Bifidobacterium longum* DSM 20219T (klinisches Isolat), *Lactobacillus acidophilus* PZ1138 (klinisches Isolat), *Lactobacillus fermentum* PZ1162 (klinisches Isolat) und *Streptococcus salivarius* spp. *termophilus* DSM20617 wurden von Ardeypharm (Deutschland) erhalten, und *Bacteroides vulgatus* DSM1447 wurde von der DSMZ (Deutsche Sammlung für Mikroorganismen und Zellkulturen) besorgt. Der *Candida albicans* Stamm 526 wurde aus Faeces isoliert, und durch das Institut der Labormedizin, Klinik am Eichert (Göppingen, Deutschland) bereitgestellt. Referenzstämme der "American Type Culture Collection (ATCC) *Escherichia coli* ATCC25922, *Staphylococcus aureus* ATCC25923 und *Bacteroides fragilis* ATCC25285 wurden durch das Institut der Labormedizin, Klinik am Eichert (Göppingen, Deutschland) bereitgestellt. Ferner wurden die Stämme *Enterococcus faecalis* ATCC29212, *Candida albicans* ATCFC 10231 und *Pseudomonas aeruginosa* ATCC27853 getestet, die unter den angegebenen ATCC-Nummern bei der American Type Culture Collection erhältlich sind.

### Peptide

Humane Beta-Defensine wurden von Peptide Institut Inc., Osaka, Japan, besorgt; Carboxy-terminale Heptapeptide und Octapeptide sowie reduziertes hBD-1 wurden chemisch synthetisiert (EMC Micro Collections Tübingen, Deutschland).

### Antimikrobielle Assays

Antimikrobielle Radialdiffusionsassays für anaerobe Bakterien wurden durchgeführt wie zuvor beschrieben (siehe Schröder et al.: "Reduction of disulfide bonds unmasks potent antimicrobial activity of human beta-defensin 1", Nature, 469: 419-423 (2011)). Kurz gesagt wurden die Bakterien anaerob kultiviert (Oxoid AnaeroGen™, England), und zwar 24 Stunden lang bei 37°C auf Columbia-Agarplatten, anschließend in flüssigem TSB ("Trypticase Soy Broth") Medium überimpft und wieder für 24 Stunden kultiviert. Anschließend wurden die bakteriellen Kulturen gewaschen und auf eine optische Dichte (OD_{620 nm}) = 0,1 verdünnt, wovon 150 µl für das Wirksamkeitsassay eingesetzt wurden. Die Inkubation erfolgte in 10 ml 10 mM Natriumphosphat mit einem pH-Wert von 7,4 mit 0,3 mg/ml TSB-Pulver und 1% (w/V) low EEO-Agarose (Agarose mit sehr niedrigem EEO-Wert) (Appli-Chem) mit 0 oder 2 mM Dithiotreitol (DTT, Sigma Aldrich), und zwar unter anaeroben Bedingungen mit 1 µg synthetischen, oxidierten hBD-1 (Peptidinstitut, Japan) oder synthetischen Peptiden über drei Stunden hinweg. Auf die Platten wurde ein Überschichtungsgel mit 6% (w/v) TSB-Pulver, 1% Agarose und 10 mM Natriumphosphatpuffer (pH-Wert 7,4 oder 5,7) mit oder ohne DTT gegeben. Nach Inkubation über 48 Stunden bei 37°C wurden die Durchmesser der Inhibierungszonen gemessen. Die Versuche wurden mindestens dreimal wiederholt.

Durchflusszytometrische antimikrobieller Assays, mit denen die Membrandepolarisierung der Bakterien und Pilze gemessen wurden, wurden wie zuvor beschrieben durchgeführt (siehe Nuding et al., "A flow cytometric assay to monitor antimicrobiol activity of defensines and cationic tissue extracts", Journal of Microbiological Methods, 65: 335-380 (2006)). Kurz gesagt wurden 1,5 x 10⁶ Zellen pro ml in 1:6 verdünnte Schaedler-Medium bei 37°C mit Peptiden in einem Endvolumen von 50 µl inkubiert. Die Defensine wurden in 0,01%-iger Essigsäure gelöst und den Bakterien-/Pilz-Suspensionen mit den angegebenen Endkonzentrationen hinzugefügt. Bakterien- oder Pilz-Suspensionen, die mit Lösungsmittel (0,01% Essigsäure) inkubiert wurden, dienten dabei als Kontrolle für die Lebensfähigkeit. Nach 90 min. wurden die Suspensionen 10 min. mit 1 mg/ml des Membranpotential-sensitiven Farbstoffs DiBAC4 (3) ([bis-(1,3-Dibutylbarbiturat)trimethan oxonol]) (Invitrogen, USA) inkubiert. Die Suspensionen wurden zentrifugiert und die Sedimente in 300 ml Phosphat-gepufferter Saline resuspendiert. Der Prozentsatz der dipolarisierten fluoreszierenden Bakterien oder Pilze in der Suspension wurde mittels eines FACSCalibur-Durchflusszytometers (Becton-Dickinson, USA) unter Verwendung der Cell Quest Software (Becton-Dickinson) bestimmt. Die Versuche wurden zweimal wiederholt und zwar jeweils in Duplikaten.

### HPLC-Analvse

Zur Analyse durch die HPLC ("High Performance Liquid Chromatography), wurden die Octapeptide mit 0,1% (v/v) Trifluoressigsäure (TFA) gemischt und mit einem Agilent 1200 System (Agilent) und einer "Synergi reversed phase" (RP) Säule (250 x 4,6 mm, 4 µm, Phenomenex, Deutschland) analysiert. Der Gradient hatte einen Anstieg von 0% B bis 12% B innerhalb 24 min. (Lösungsmittel A, Wasser + 0,18% (v/v) TFA; Lösungsmittel B, Acetonitril + 0,15% (v/v) TFA) bei 25°C und 0,8 ml/min.

### Ionen-Inhibierungsassay

0,25 µg/ml der Peptide oder Defensine wurden mit 4,5 mM NaCl, Magnesiumchlorid MgCl₂, Eisenchlorid FeCl₂, Zinkchlorid ZnCl₂ oder Zinksulfat ZnSO₄ 45 min. lang bei Raumtemperatur inkubiert. Anschließend wurde die Mischung in Radialdiffusionsassays auf ihre antimikrobielle Aktivität gegen *Bifidobacterium adolescentis* und Escherichia coli analysiert. Die Versuche wurden mindestens dreimal wiederholt.

### Ergebnisse

Schröder et al. ("Reduction of disulfid bonds unmasks potent antimicrobial activity of human beta-defensine 1", Nature, 469: 419-423 (2011)) haben kürzlich gezeigt, dass menschliches Beta-Defensin 1 eine erhöhte antimikrobielle Aktivität unter reduzierenden Bedingungen zeigt.

Vorliegend wurde hBD-1 bzw. dessen antimikrobielle Aktivität weiter untersucht. Hierzu wurde die antimikrobielle Aktivität der drei menschlichen Beta-Defensine hBD-1, 2 und 3 gegenüber kommensalen Bakterien der menschlichen Darmflora getestet, und zwar unter Standardbedingungen (pH 7,4) und leicht sauren Bedingungen (pH 5,7), wobei unter beiden Bedingungen auch reduzierende Bedingungen getestet wurden, in dem 2 mM des chemischen reduzierenden Agens Dithiothreitol (DTT) dem Wachstumsmedium zugegeben wurde. Dabei zeigte sich (Daten nicht gezeigt), dass bei den meisten Bakterien unter Standardbedingungen die Aktivität der Beta-Defensine am höchsten war, mit der Ausnahme von hBD-1, das unter diesen Bedingungen weitgehend inaktiv war, und durch Reduktion aktiv wurde. Diese Aktivierung konnte jedoch nicht bei einem pH-Wert von 5,7 beobachtet werden. Im Gegensatz dazu erwies sich hBD-2 durch eine Reduktion nicht beeinflussbar, wohingegen eine Veränderung des pH-Werts einen sehr negativen Effekt auf dessen antimikrobielle Aktivität hatte.

hBD-3 hatte in den meisten Fällen die stärkste Aktivität gegen die getesteten kommensalen im Vergleich zu den beiden anderen Defensinen.

Zusammengefasst lässt sich sagen, dass Faktoren des umgebenden Milieus, wie beispielsweise das Redox-Potential und der pH-Wert, die antimikrobielle Aktivität der Beta-Defensine gegen kommensale Darmbakterien modulieren kann. Diese Modulation scheint jedoch für einzelne Defensin-Bakterium-Beziehungen spezifisch zu sein, und korreliert weder mit dem Gram-Status noch mit dem Genus des Bakteriums.

Versuche mit einem Heptapeptid, das die sieben terminalen Aminosäuren von hBD-1 darstellt, das bereits eine antimikrobielle Aktivität gegen *Bifidobacterium adolescentis* zeigt, haben gezeigt, dass das Carboxy-terminale Heptapeptid des Wildtyps die höchste Aktivität gegen *Bifidobacterium adolescentis* hatte, wohingegen Peptide mit einer umgekehrten Aminosäuresequenz weniger aktiv waren. Durch einen Austausch der Cysteinreste mit Alanin wurde Aktivität vollständig unterbunden. Der isolierte Amino-Terminus von hBD-1 war inaktiv.

Vorliegend wurde als nächstes das erfindungsgemäße Peptid, ein Octapeptid, das die acht terminalen Aminosäuren des Carboxy-Terminus von hBD-1 umfasst, getestet: Dieses zeigte eine stärkere antimikrobielle Aktivität als das zuvor getestete Heptapeptid (siehe Fig. 1). Wenn entweder Cys₆ oder Cys₇ ausgetauscht wurde, wurde die Aktivität stark gedrosselt in Bezug auf *Bifidobacerium adolescensis,* und zwar im gleichen Ausmaß, wohingegen ein Austausch beider Cysteine die Aktivität vollständig zum Erliegen brachte. Im Gegensatz zu dem zuvor getesteten Heptapeptid hatte das Octapeptid auch eine antimikrobielle Aktivität gegenüber *Escherichia coli* (siehe Fig. 1b). Überraschenderweise steigerte hier der Austausch von Cys₆ oder Cys₇ durch Alanin die antimikrobielle Aktivität, wohingegen der Austausch beider Cysteine wieder die antimikrobielle Aktivität beinahe vollständig ausschaltete.

Zur Optimierung des Octapeptids bzw. zur Verbesserung dessen Stabilität gegenüber Proteasen wurden in einem nächsten Schritt der Amino-Terminus des Octapeptids durch Acetylierung und der Carboxyterminus mit Amidierung stabilisiert. Während sich die Aktivität gegenüber *Escherichia coli* nicht signifikant im Vergleich zum Wildtyp-Peptid unterschied, steigerte sich die Aktivität gegenüber *Bifidobacterium adoslescentis* stark (siehe Fig. 1c).

Mittels des neu identifizierten und bereitgestellten Octapeptids wird ein einfach und günstig herzustellendes Peptid mit antibiotischen Wirkungen bereitgestellt, das als therapeutisches Mittel eingesetzt werden kann. Sowohl die modifizierten als auch die unmodifizierten Peptide wurden daher auf ihre Fähigkeit untersucht, (opportunistische) pathogene Mikroorganismen abzutöten. Hierzu wurden durchflusszytometrische Assays durchgeführt, (siehe Fig. 2), aus denen sich gezeigt hat, dass die Wirksamkeit des Wildtyp-Octapeptids sowie des Alanin-mutierten Peptids und des Wildtyp Heptapeptids lediglich marginal in den meisten Fällen war. Im Gegensatz dazu hatte das modifizierte Octapeptid eine herausragende Aktivität gegen die pathogene Mikroorganismen *Staphylococcus aureus* und *Candida albicans,* nicht jedoch gegenüber *Escherichia coli* und *Bacteroides fragilis.*

Damit zeigte sich, dass durch die Stabilisierung der Termini nicht nur die antimikrobielle Aktivität gegenüber dem kommensalen Darmbakterium *Bifidobacterium adolescentis* erhöht wird, sondern auch gegenüber mindestens zwei pathogenen Mikroorganismen klinischer Relevanz.

In weiteren Versuchen wurde eine reversed phase HPLC-Analyse durchgeführt, um die Hydrophobizität der getesteten Peptide zu untersuchen. (Daten nicht gezeigt). Das modifizierte Octapeptid eluierte als letztes von der Säule, was auf die höchste Hydrophobizität hindeutete; davor erfolgte die Elution des Wildtyp-Peptids, der einzelnen Alanin-Aminosäure-Austauschvarianten und der Doppel-Alanin-Aminosäure-Austauschvariante (Daten nicht gezeigt).

Um die Rolle der Ladung und der loneninteraktionen weiter zu untersuchen, wurden oxidiertes und reduziertes hBD-1 sowie das Wildtyp und das modifizierte Octapeptid mit monovalenten und divalenten Kationen inkubiert (Daten nicht gezeigt). Bezüglich *Escherichia coli* wurde die Aktivität des vollständigen Defensins durch Vorinkubation mit Magnesiumchlorid oder Eisenchlorid vollständig heruntergefahren, wohingegen die Aktivität der Carboxy-terminalen Peptide stark inhibiert wurde, jedoch noch detektierbar war nach Vorinkubation mit diesen Metallionen. Im Gegensatz dazu beeinflusste NaCl die antimikrobielle Aktivität gegenüber *E. coli* nicht.

Bei der Untersuchung von *Bifidobacterium adolescentis* zeigte sich wieder, dass die Vorinkubation mit Natriumchlorid keinen starken Effekt auf die Aktivität hatte, wohingegen Eisenchlorid, Zinkchlorid und Zinksulfat die Aktivität vollständig abschafften oder stark reduzierten. Im Gegensatz zu *E. coli* beeinflusste eine Inkubation mit Magnesiumchlorid die antibiotische Aktivität gegen *Bifidobacterium* nicht.

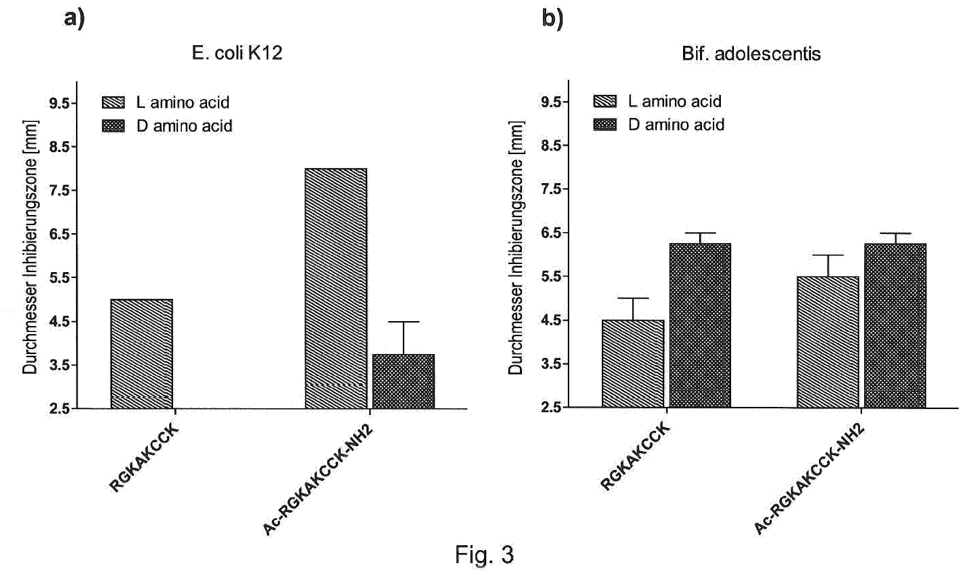

in weiteren Versuchen wurde ein Octapeptid mit der Sequenz RGKAKCCK (SEQ ID Nr. 1) bestehend aus D-Aminosäuren - bis auf Glycin - im Vergleich zu einem Octapeptid mit der Sequenz RGKAKCCK (SEQ ID Nr. 1) bestehend aus L-Aminosäuren untersucht, und zwar mit modifizierten oder unmodifizierten Termini (Fig. 3). Gegenüber *E. coli* K12 (siehe Fig. 3a) zeigte das aus D-Aminosäuren bestehende Peptid mit modifizierten Termini (N-Terminus: Acetylierung; C-Terminus: Amidierung) eine schwächere Aktivität, wohingegen dieses Peptid, sowohl in der unmodifizierten Form also auch der modifizierten Form, gegenüber *Bifidobacterium adolescentis* (siehe Fig. 3b) eine gegenüber dem aus L-Aminosäuren bestehenden Peptid erhöhte Aktivität aufwies.

Diese Daten zeigen also insgesamt, dass die Interaktion zwischen Peptid und Kationen nicht nur auf einer positiven Ladung basiert, sondern dass vielmehr spezifische Ionen die Aktivität gegen bestimmte Bakterien beeinflussen können.

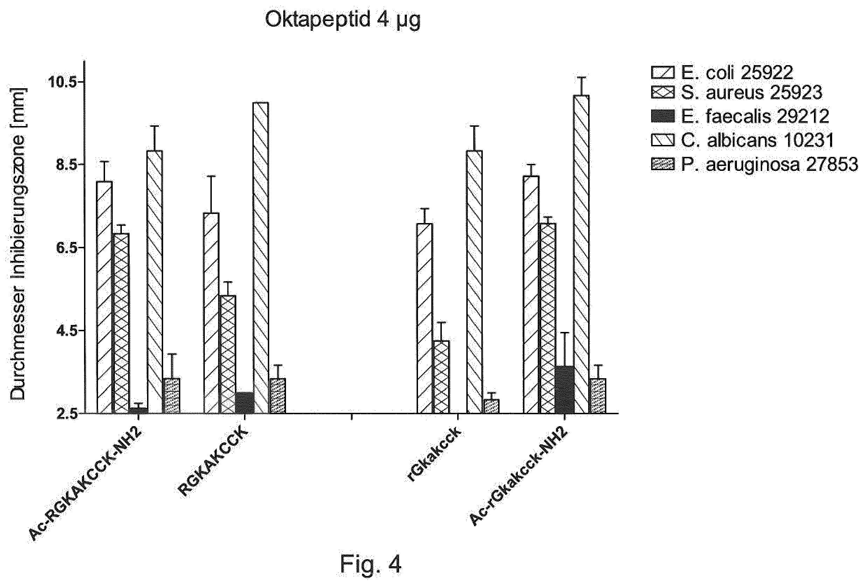

Die Aktivität der erfindungsgemäßen Octapeptide gegen pathogene Bakterien und Pilze wurde auch in weiteren Radial-Diffusionsassays bestätigt: getestet wurde die Aktivität gegen die Stämme *Escherichia coli* 25922, *Staphylococcus aureus* 25923, *Enterococcus faecalis* 29212, *Candida albicans* 10231 und *Pseudomonas aeruginosa* 27853 (siehe Fig. 4), wobei einerseits das Octapeptid mit der Sequenz RGKAKCCK (SEQ ID Nr. 1) bestehend entweder aus L-Aminosäuren (siehe Fig. 4, die fünf Säulen links im Diagramm) oder bestehend aus D-Aminosäuren - bis auf Glycin - (siehe Fig. 4, die fünf Säulen rechts im Diagramm) eingesetzt wurde, und zwar jeweils einmal mit modifizierten und einmal mit unmodifizierten Termini (N-Terminus: Acetylierung; C-Terminus: Amidierung). Hier zeigte sich - neben einer herausragenden Aktivität gegen *Escherichia coli* und *Staphylococcus aureus* - insbesondere auch eine ausgezeichnete Aktivität gegenüber *Candida albicans* aller Varianten des getesteten Octapeptids.

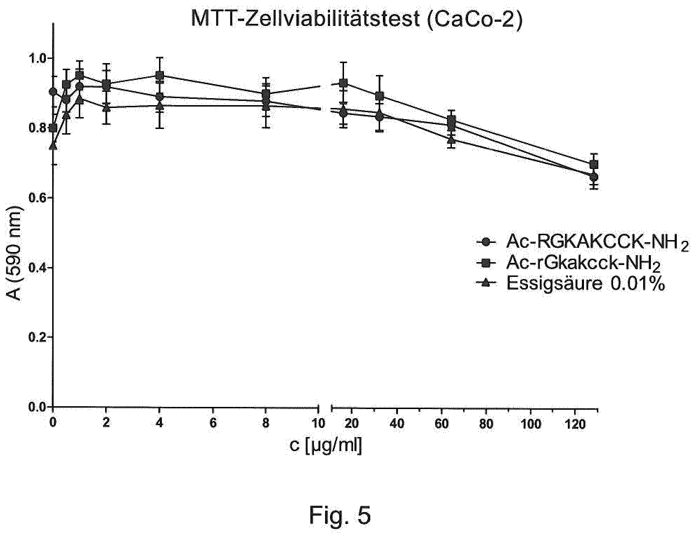

In weiteren MTT-((3-[4,5-Dimethylthiazol-2-yl]-2,5-diphenyltetrazolium bromid; Thiazolylblau) Versuchen wurde noch die Zelltoxizität der terminal stabilisierten Octapeptide untersucht, und zwar gegenüber der humanen Zelllinie CaCo-2 (ATCC HTB-37), mit ansteigenden Konzentrationen der jeweiligen Octapeptide (SEQ ID Nr. 1; L- oder D- Aminosäuren (bis auf Glycin) mit modifizierten Termini: N-Terminus: Acetylierung und C-Terminus: Amidierung), im Vergleich zu ansteigenden Konzentrationen an 0,01% Essigsäure. Die Ergebnisse hierzu sind in Fig. 5 wieder gegeben, wobei sich zeigte, dass die stabilisierten Octapeptide keine Zelltoxizität besaßen, die über diejenige des Lösungsmittels 0,01% Essigsäure hinausgeht. Damit sind die erfindungsgemäßen Octapeptide auch geeignet, in therapeutischen Anwendungen eingesetzt zu werden.

Die oben ausgeführten Ergebnisse und Daten zeigen jedoch klar, dass das vorliegend erstmals bereitgestellte Octapeptid, und zwar in Wildtyp-Form, mit einem Aminosäurenaustausch, und/oder in stabilisierter Form ein herausragendes Mittel mit antibiotischer Wirksamkeit ist. Diese Ergebnisse sind überraschend und waren aufgrund des bisher verfügbaren Standes der Technik nicht zu erwarten.

## Patentansprüche

1. Peptid, das antimikrobielle Aktivität aufweist, und einen C- und einen N-Terminus aufweist, und das aus acht aufeinander folgenden Aminosäuren besteht, wobei das Peptid eine Sequenz mit der folgenden Formel I besitzt:
Ter₁-X₁-B₁-X₂-B₂-X₃-Z₁-Z₂-X₄-Ter₂, (Formel I)
in welcher
Ter₁ die freie N-terminale Aminogruppe der N-terminalen Aminosäure X₁ ist, oder eine modifizierte N-terminale Aminogruppe;
X₁, X₂ und X₃ jeweils gleich oder unterschiedlich sind und unabhängig voneinander jeweils ausgewählt sind aus einer Aminosäure mit einer basischen Seitenkette, vorzugsweise ausgewählt sind aus einem der folgenden: Arginin, Lysin, 6-Hydroxylysin, Homoarginin, 2,4-Diaminobuttersäure, [beta]-Homoarginin, D-Arginin, Arginal, 2-Amino-3-guanidinopropionsäure, Nitroarginin, N-Methyl-Arginin, [epsilon]-N-Methyllysin, allo-Hydroxylysin, 2,3-Diaminopropionsäure, 2,2'-Diaminopimelinsäure, Ornithin, sym-Dimethylarginin, asym-Dimethylarginin,
B₁ und B₂ gleich oder unterschiedlich sind und jeweils eine Aminosäure mit einer aliphatischen oder basischen Seitenkette sind, und vorzugsweise aus Alanin oder Glycin ausgewählt sind,
Z₁ und Z₂ jeweils Cystein und Alanin sind, und
Ter₂ die freie C-terminale Carboxylgruppe der C-terminalen Aminosäure X₄ ist, oder eine modifizierte C-terminale Carboxylgruppe.

2. Peptid, oder Derivat davon, nach Anspruch 1, **dadurch gekennzeichnet, dass** das Peptid ausgewählt ist aus einer der SEQ ID Nr. 2, 3, 5 und 6.

3. Peptid nach einem der Ansprüche 1 oder 2, **dadurch gekennzeichnet, dass** es aus D- oder L-Aminosäuren oder Mischungen davon besteht.

4. Peptid nach einem der Ansprüche 1 bis 3, **dadurch gekennzeichnet, dass** das Peptid am C- und/oder N-Terminus durch eine Acetylierung, Amidierung, Formylierung, Phosphorylierung, modifiziert ist.

5. Peptid nach einem der Ansprüche 1 bis 4 zur Verwendung als Antibiotikum.

6. Peptid zur Verwendung nach Anspruch 5, zur Behandlung und/oder Prophylaxe von entzündlichen oder infektiösen Erkrankungen, die durch Mikroorganismen ausgelöst werden.

7. Peptid zur Verwendung nach Anspruch 6, **dadurch gekennzeichnet, dass** die entzündliche oder infektiöse Erkrankung durch einen Mikroorganismus verursacht wird, der ein Bakterium, ein Virus oder ein Hefepilz ist.

8. Peptid zur Verwendung nach einem der Ansprüche 5 bis 7, **dadurch gekennzeichnet, dass** die entzündliche oder infektiöse Erkrankung durch ein Mikroorganismus verursacht wird, der ausgewählt ist aus *Bifidobacterium* sp., *Lactobacillus* sp., *Escherichia coli, Streptococcus sp., Staphylococcus sp., Bacteroides sp., Candida sp., Pseudomonas sp., Propionibakterium sp., Treponema sp.*

9. Peptid zur Verwendung nach einem der Ansprüche 5 bis 8, **dadurch gekennzeichnet, dass** die entzündliche oder infektiöse Erkrankung ausgewählt ist aus chronisch entzündlichen Darmerkrankungen, entzündlichen Erkrankungen des Mund-/Rachenraumes, Lungenerkrankungen, Erkrankungen des Urogenitaltraktes, Erkrankungen der Bauchspeicheldrüse, Erkrankungen des weiblichen Reproduktionstraktes, Erkrankungen oder Verletzungen oder Verbrennungen der Haut.

10. Pharmazeutische Zusammensetzung, enthaltend zumindest ein Peptid nach einem der Ansprüche 1 bis 4 sowie einen pharmazeutisch akzeptierbaren Träger.

11. Polynucleotid, codierend für ein Peptid nach einem der Ansprüche 1 bis 4

12. Verwendung des Polypeptids nach einem der Ansprüche 1 - 4 in einem Desinfektions- oder Reinigungsmittel.

## Claims

1. Peptide which exhibits antimicrobial activity and has a C-terminus and an N-terminus and which consists of eight successive amino acids, the peptide having a sequence having the following formula I:
Ter₁-X₁-B₁-X₂-B₂-X₃-Z₁-Z₂-X₄-Ter₂, (formula I)
in which
Ter₁ is the free N-terminal amino group of the N-terminal amino acid X₁ or a modified N-terminal amino group;
X₁, X₂ and X₃ are each identical or different and are each independently selected from an amino acid having a basic side chain, are preferably selected from one of the following: arginine, lysine, 6-hydroxylysine, homoarginine, 2,4-diaminobutyric acid, [beta]-homoarginine, D-arginine, arginal, 2-amino-3-guanidinopropionic acid, nitroarginine, N-methylarginine, [epsilon]-N-methyllysine, allo-hydroxylysine, 2,3-diaminopropionic acid, 2,2'-diaminopimelic acid, ornithine, symdimethylarginine, asym-dimethylarginine,
B₁ and B₂ are identical or different and are each an amino acid having an aliphatic or basic side chain, and are preferably selected from alanine or glycine,
Z₁ and Z₂ are each cysteine or alanine, and
Ter₂ is the free C-terminal carboxyl group of the C-terminal amino acid X₄ or a modified C-terminal carboxyl group.

2. Peptide, or derivative thereof, according to Claim 1, **characterized in that** the peptide is selected from any of SEQ ID No. 2, 3, 5 and 6.

3. Peptide according to either of Claims 1 and 2, **characterized in that** it consists of D- or L-amino acids or mixtures thereof.

4. Peptide according to any of Claims 1 to 3, **characterized in that** the peptide is modified on the C-terminus and/or N-terminus by an acetylation, amidation, formylation, phosphorylation.

5. Peptide according to any of Claims 1 to 4 for use as antibiotic.

6. Peptide for the use according to Claim 5, for the treatment and/or prophylaxis of inflammatory or infectious diseases which are caused by microorganisms.

7. Peptide for the use according to Claim 6, **characterized in that** the inflammatory or infectious disease is caused by a microorganism which is a bacterium, a virus or a yeast fungus.

8. Peptide for the use according to any of Claims 5 to 7, **characterized in that** the inflammatory or infectious disease is caused by a microorganism which is selected from *Bifidobacterium* sp., *Lactobacillus* sp., *Escherichia coli, Streptococcus* sp., *Staphylococcus* sp., *Bacteroides* sp., *Candida* sp., *Pseudomonas* sp., *Propionibacterium* sp., *Treponema* sp.

9. Peptide for the use according to any of Claims 5 to 8, **characterized in that** the inflammatory or infectious disease is selected from chronically inflammatory bowel diseases, inflammatory diseases of the oral cavity/pharynx, pulmonary diseases, diseases of the urogenital tract, diseases of the pancreas, diseases of the female reproductive tract, diseases of or injuries to or burns to the skin.

10. Pharmaceutical composition containing at least one peptide according to any of Claims 1 to 4 and also a pharmaceutically acceptable carrier.

11. Polynucleotide coding for a peptide according to any of Claims 1 to 4.

12. Use of the polypeptide according to any of Claims 1-4 in a disinfectant or cleaning agent.

## Revendications

1. Peptide, qui présente une activité antimicrobienne, et comprend une terminaison C et une terminaison N, et qui est constitué par huit acides aminés successifs, le peptide présentant une séquence de la formule I suivante :
Ter₁-X₁-B₁-X₂-B₂-X₃-Z₁-Z₂-X₄-Ter₂ (Formule I)
dans laquelle
Ter₁ est le groupe amino N-terminal libre de l'acide aminé N-terminal X₁, ou un groupe amino N-terminal modifié ;
X₁, X₂ et X₃ sont chacun identiques ou différents, et sont chacun choisis indépendamment les uns des autres parmi un acide aminé comprenant une chaîne latérale basique, de préférence sont choisis parmi un des suivants : arginine, lysine, 6-hydroxylysine, homoarginine, acide 2,4-diaminobutyrique, [bêta]-homoarginine, D-arginine, arginal, acide 2-amino-3-guanidinopropionique, nitroarginine, N-méthyl-arginine, [epsilon]-N-méthyllysine, allo-hydroxylysine, acide 2,3-diaminopropionique, acide 2,2'-diaminopimélique, ornithine, sym-diméthylarginine, asym-diméthylarginine, B₁ et B₂ sont identiques ou différents, et sont chacun un acide aminé comprenant une chaîne latérale aliphatique ou basique, et sont de préférence choisis parmi l'alanine ou la glycine,
Z₁ et Z₂ sont respectivement la cystéine et l'alanine, et Ter₂ est le groupe carboxyle C-terminal libre de l'acide aminé C-terminal X₄, ou un groupe carboxyle C-terminal modifié.

2. Peptide, ou dérivé de celui-ci, selon la revendication 1, **caractérisé en ce que** le peptide est choisi parmi une des SEQ ID N° 2, 3, 5 et 6.

3. Peptide selon l'une quelconque des revendications 1 ou 2, **caractérisé en ce qu'**il est constitué par des acides D- ou L-aminés ou des mélanges de ceux-ci.

4. Peptide selon l'une quelconque des revendications 1 à 3, **caractérisé en ce que** le peptide est modifié à la terminaison C et/ou N par une acétylation, une amidation, une formylation, une phosphorylation.

5. Peptide selon l'une quelconque des revendications 1 à 4, destiné à une utilisation en tant qu'antibiotique.

6. Peptide destiné à une utilisation selon la revendication 5, pour le traitement et/ou la prophylaxie de maladies inflammatoires ou infectieuses, qui sont déclenchées par des microorganismes.

7. Peptide destiné à une utilisation selon la revendication 6, **caractérisé en ce que** la maladie inflammatoire ou infectieuse est causée par un microorganisme qui est une bactérie, un virus ou une levure.

8. Peptide destiné à une utilisation selon l'une quelconque des revendications 5 à 7, **caractérisé en ce que** la maladie inflammatoire ou infectieuse est causée par un microorganisme qui est choisi parmi *Bifidobacterium sp., Lactobacillus sp., Escherichia coli, Streptococcus sp., Staphylococcus sp., Bacteroides sp., Candida sp., Pseudomonas sp., Propionibakterium sp., Treponema sp.*

9. Peptide destiné à une utilisation selon l'une quelconque des revendications 5 à 8, **caractérisé en ce que** la maladie inflammatoire ou infectieuse est choisie parmi les maladies intestinales inflammatoires chroniques, les maladies inflammatoires de la bouche/du pharynx, les maladies pulmonaires, les maladies de l'appareil urogénital, les maladies du pancréas, les maladies de l'appareil reproducteur féminin, les maladies ou blessures ou brûlures de la peau.

10. Composition pharmaceutique, contenant au moins un peptide selon l'une quelconque des revendications 1 à 4, ainsi qu'un support pharmaceutique acceptable.

11. Polynucléotide, codant pour un peptide selon l'une quelconque des revendications 1 à 4.

12. Utilisation du polypeptide selon l'une quelconque des revendications 1 à 4 dans un agent désinfectant ou nettoyant.


### SEQUENZ PROTOKOLL

<110> Robert Bosch GmbH
<120> Antimikrobielle Peptide
<130> R. 342354
<160> 12
<170> PatentIn version 3.3
<210> 1
   <211> 8
   <212> PRT
   <213> Künstliche Sequenz
<220>
   <223> Synthetisch hergestelltes Peptid, umfassend Sequenzen des C-Terminus von hBD-1
<400> 1
   Arg Gly Lys Ala Lys Cys Cys Lys
   1               5
<210> 2
   <211> 8
   <212> PRT
   <213> Künstliche Sequenz
<220>
   <223> Synthetisches Peptid mit antimikrobieller Aktivität
<400> 2
   Arg Gly Lys Ala Lys Cys Ala Lys
   1               5
<210> 3
   <211> 8
   <212> PRT
   <213> Künstliche Sequenz
<220>
   <223> Synthetisches Peptid mit antimikrobieller Aktivität
<400> 3
   Arg Gly Lys Ala Lys Ala Cys Lys
   1               5
<210> 4
   <211> 8
   <212> PRT
   <213> Künstliche Sequenz
<220>
   <223> Synthetisches Peptid mit modifizierten Termini
<220>
   <221> MOD_RES
   <222> (1)..(1)
   <223> ACETYLATION
<220>
   <221> MOD_RES
   <222> (8)..(8)
   <223> AMIDATION
<400> 4
   Arg Gly Lys Ala Lys Cys Cys Lys
   1               5
<210> 5
   <211> 8
   <212> PRT
   <213> Künstliche Sequenz
<220>
   <223> Synthetisches Peptid mit modifizierten Termini
<220>
   <221> MOD_RES
   <222> (1)..(1)
   <223> ACETYLATION
<220>
   <221> MOD_RES
   <222> (8)..(8)
   <223> AMIDATION
<400> 5
   Arg Gly Lys Ala Lys Cys Ala Lys
   1               5
<210> 6
   <211> 8
   <212> PRT
   <213> Künstliche Sequenz
<220>
   <223> Synthetisches Peptid mit modifizierten Termini
<220>
   <221> MOD_RES
   <222> (1)..(1)
   <223> ACETYLATION
<220>
   <221> MOD_RES
   <222> (8)..(8)
   <223> AMIDATION
<400> 6
   Arg Gly Lys Ala Lys Ala Cys Lys
   1               5
<210> 7
   <211> 8
   <212> PRT
   <213> Künstliche Sequenz
<220>
   <223> Synthetisches Peptid
<400> 7
   Arg Gly Lys Ala Lys Ala Ala Lys
   1               5
<210> 8
   <211> 7
   <212> PRT
   <213> Künstliche Sequenz
<220>
   <223> synthetisches Peptid
<400> 8
   Gly Lys Ala Lys Cys Cys Lys
   1               5
<210> 9
   <211> 7
   <212> PRT
   <213> Künstliche Sequenz
<220>
   <223> Synthetisches Peptid
<400> 9
   Gly Lys Ala Lys Ala Ala Lys
   1               5
<210> 10
   <211> 7
   <212> PRT
   <213> Künstliche Sequenz
<220>
   <223> Synthetisches Peptid
<400> 10
   Lys Cys Cys Lys Ala Lys Gly
   1               5
<210> 11
   <211> 7
   <212> PRT
   <213> Künstliche Sequenz
<220>
   <223> Synthetisches Peptid
<400> 11
   Cys Lys Lys Cys Lys Ala Gly
   1               5
<210> 12
   <211> 7
   <212> PRT
   <213> Künstliche Sequenz
<220>
   <223> Synthetisches Peptid
<400> 12
   Asp His Tyr Asn Cys Val Ser
   1               5